Europäisches Patentamt

(19) **European Patent Office**

Office européen des brevets

(11) Numéro de publication: **0 045 237**
**B1**

## (12) FASCICULE DE BREVET EUROPEEN

(45) Date de publication du fascicule du brevet:
27.06.84

(51) Int. Cl.³: **C 07 G 7/00, A 61 K 37/02, A 61 K 35/74**

(21) Numéro de dépôt: **81401114.4**

(22) Date de dépôt: **09.07.81**

(54) **Nouvelle substance immuno-suppressive, son procédé d'isolement et son application en thérapeutique.**

(30) Priorité: **28.07.80 FR 8016582**

(43) Date de publication de la demande:
**03.02.82 Bulletin 82/5**

(45) Mention de la délivrance du brevet:
**27.06.84 Bulletin 84/26**

(84) Etats contractants désignés:
**AT BE CH DE FR GB IT LI LU NL SE**

(73) Titulaire: **BERRI BALZAC Société dite:, 42, Rue Rouget-de-Lisle, F-92151 Suresnes (FR)**

(72) Inventeur: **Arala-Chaves, Mario, Rua Filipe da Mata 64-4, Lisbonne (PT)**

(74) Mandataire: **Polus, Camille et al, c/o Cabinet Lavoix 2, Place d'Estienne d'Orves, F-75441 Paris Cedex 09 (FR)**

(56) Documents cités:
CHEMICAL ABSTRACTS, vol. 91, no. 25, 17 décembre 1979, réf. 209098m page 497 Columbus, Ohio, US MARIO P. ARALA-CHAVES et al.: "Evidence for the synthesis and release of strongly immunosuppressive, noncytotoxic substances by Streptococcus intermedius"
CHEMICAL ABSTRACTS, vol. 88, no. 21, 22 mai 1978, réf.: 150443c, page 431 Columbus, Ohio, US C.M. CUNNINGHAM et al.: "Suppression of antibody response by group A streptococcal pyrogenic exotoxin and characterization of the cells involved"
CHEMICAL ABSTRACTS, vol. 88, no. 15, 10 avril 1978, réf.: 100082p, page 129 Columbus, Ohio, US C.M. CUNNINGHAM et al.: "Alteration of clearance function by group A streptococcal pyrogenic exotoxin and its relation to suppression of the antibody response"

(56) Documents cités: (suite)
CHEMICAL ABSTRACTS, vol. 82, no. 15, 14 avril 1975, réf.: 96348m. page 311 Columbus, Ohio, US H. FINGER et al.: "Immunosuppressant present in cytoplasmic preparations of beta-gemolytic streptococci"
CHEMICAL ABSTRACTS, vol. 79, no. 5 6 août 1973, réf.: 30394e, page 323 Columbus, Ohio, US E.E. HANNA et al.: "Enhanced immune response after immunosuppression by streptococcal pyrogenic exotoxin"
CHEMICAL ABSTRACTS, vol. 76, no. 11, 13 mars 1972, réf.: 57539f, page 308 Columbus, Ohio, US A.H. MALAKIAN et al.: "Biological characterization of an immunosuppressant from group A streptococci"
CHEMICAL ABSTRACTS, vol. 77, no. 11, 11 septembre 1972, réf.: 73556k, page 348 Columbus, Ohio, US H. GAUMER et al.: "Differential susceptibility of mouse lymphocytes to an immunosuppressant from group A streptococci"

**Description**

La présente invention est relative à une nouvelle substance immuno-suppressive, à son procédé d'isolement et à ses applications en thérapeutique.

Mario P. Arala-Chaves et coll. (J. Clin. Invest. vol. 64, oct. 1979, p. 871—883) ont montré que des extraits bruts de Streptococcus intermedius avaient une puissante activité immuno-suppressive.

Le Demandeur a maintenant réussi à isoler la substance responsable de cette activité immuno-suppressive et a ainsi permis son application en thérapeutique.

La nouvelle substance immuno-suppressive selon l'invention est une protéine possédant les propriétés physico-chimiques suivantes:

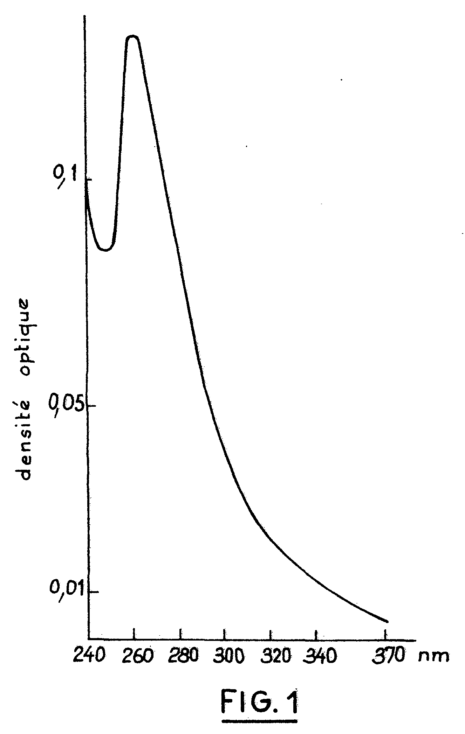

1 — Elle possède un point isoélectrique de 4,25.
2 — Elle possède un spectre d'absorption ultraviolet, comme représenté sur la fig. 1, présentant un maximum à 260 nm.
3 — Elle possède une masse moléculaire d'environ 90 000 daltons.
4 — Elle est colorable au bleu de Coomasie; elle n'est pas colorable au bleu de méthylène ni au PAS.
5 — Elle est résistante à la désoxyribonucléase, à la ribonucléase A et à la neuraminidase.

Elle est sensible aux $\alpha$, $\gamma$ et $\delta$ chymotrypsines et à la trypsine.

En outre, elle est sensible à la chaleur: elle est totalement détruite à 70°C après 30 min, partiellement détruite à 56°C après 30 min et très légèrement détruite après incubation à 37°C pendant 24 heures.

De plus, elle adhère au Sephadex® et à l'acétate de cellulose et l'on pense qu'elle contient de la phénylalanine parmi ses constituants principaux.

Il a également été constaté que cette substance forme des complexes avec d'autres constituants biologiquement inactifs présents dans les extraits non purifiés.

La substance selon l'invention peut être obtenue à partir de produits sécrétés par certains microorganismes et notamment par Streptococcus intermedius.

Les produits sécrétés par ces microorganismes peuvent être notamment obtenus selon la technique déjà décrite par Mario P. Arala-Chaves et coll. (référence déjà citée), qui consiste à effectuer une culture du microorganisme, séparément du milieu de culture, sur une membrane de dialyse, à laver la membrane, à éliminer les microorganismes du liquide de lavage par centrifugation et à concentrer le liquide débarrassé des microorganismes par ultracentrifugation ou dyalise sous vide.

La séparation de la substance selon la présente invention de ces extraits peut être effectuée comme suit: on soumet les extraits à une isoélectrofocalisation préparative sur gradient de saccharose à pH 3,5—6 on sépare les fractions présentant une activité immuno-suppressive, on soumet ces fractions à une isoélectrofocalisation préparative sur gradient de saccharose à pH 4—5 et l'on isole la fraction présentant une activité immuno-suppressive.

L'isoélectrofocalisation préparative peut être mise en oeuvre selon une technique basée sur les principes décrits par A. Winter et C. Karisson (1976) Preparative electrofocusing in density gradients. LKB Application Note 219, page 1—15.

Les fractions contenant la substance selon l'invention ont été isolées par mesure de l'activité immuno-suppressive selon les techniques suivantes:

a) Captage de [³H]-thymidine par des cellules mononucléaires humaines stimulées par PHA

Des cellules mononucléaires de sang provenant de 6 donneurs différents sont obtenues à partir de sang défibriné après centrifugation sur un gradient Ficoll-métrizoate de sodium selon la méthode de Boyum (Scand J. Clin. Lab. Invest. 21:7—109).

Ces cellules sont cultivées selon la méthode de Du Bois et coll. (Tissue Antigens 3: 402—409) dans des tubes de Nunc. On utilise comme milieu de culture du milieu essentiel minimal (MEM de Gibco Diagnostics) complété avec 20% de sérum AB humain frais inactivé par la chaleur et des antibiotiques (100 U/ml de pénicilline, 100μg/ml de streptomycine).

Le milieu est tamponné avec du tris-HCl.

On utilise de la phytohémagglutinine (PHA, Gibco) comme stimulant de la concentration finale de 1/40.

Les fractions à tester sont ajoutées, soit au début des cultures, soit après l'addition du stimulant.

Deux jours après le début de la culture, on ajoute aux cultures 0,5 μci de [³H]-thymidine, 24 heures avant la récolte. Les cultures sont récoltées 24 heures après.

On détermine l'activité immuno-suppressive par la réduction du captage de [³H]-thymidine dans ces cultures par rapport à des cultures témoins stimulées par PHA.

b) Captage de [³H]-thymidine par des cellules mononucléaires humaines stimulées par MLC

Cette technique, qui est une variante de la précédente, utilise une culture de lymphocytes mixte (MLC).

On réalise une culture de lymphocytes mixte bidirectionnelle avec $1,5 \cdot 10^5$ cellules mononucléaires de deux donneurs humains HLA-incompatibles.

On ajoute les fractions à tester au début des cultures à des dilutions de 1/200 à 1/25 600.

Cinq jours après le début de la culture on ajoute aux cultures 0,5 µ Ci de [³H]-thymidine. Les cultures sont récoltées 24 heures aprés.

c) Immunisation de cellules mononucléaires humaines contre SRBC, consistant à inhiber la génération in vitro de cellules mononucléaires de sang périphérique humain capable de produire des anticorps contre les globules rouges de mouton

On a utilisé la méthode déjà décrite par Mario P. Arala-Chaves et coll. (référence déjà citée).

d) Essai in vivo chez la souris

On a utilisé la méthode précédemment décrite par Mario P. Arala-Chaves et coll. (référence déjà citée).

On illustrera ci-après plus spécifiquement l'isolement de la substance selon l'invention à partir de Streptococcus intermedius.

On cultive Streptococcus intermedius dans des conditions anaérobies sur des disques de membranes stériles de dialyse (laissant passer les substances jusqu'à une masse moléculaire de 12 000 à 14 000) ayant un diamètre de 100 mm disposées à la surface d'un milieu de culture tryptone-glucose-agar (contenant 20 g tryptone, 5 g de glucose, 4 g de $K_2HPO_4$, 1 g de $KH_2PO_4$, 2 g de NaCl, 250 mg de $MgSO_4 \cdot 7 H_2O$, 17 mg de $MnSO_4$ et 15 g d'agar dans un litre d'eau distillée).

On étale sur la membrane de dialyse 0,1 ml de suspension bactérienne contenant $10^8$ cellules par ml à l'aide d'un tampon d'ouate.

La culture est effectuée à 37°C pendant 24 heures. On retire ensuite les membranes et on les laisse avec un volume minimal de tampon phosphate de potassium 0,05 M, pH 7,5. On centrifuge à 29 000 g pendant 15 mn le liquide de lavage pour séparer les microorganismes. On concentre le surnageant environ 10 fois par ultrafiltration sous pression positive avec un filtre Amicon PM 10.

Cette dernière opération permet en outre la séparation des éléments nutritifs dialysables provenant du milieu de culture. On obtient des préparations concentrées contenant 1490 µg/ml de protéine.

Les préparations concentrées de produits secrétés par Streptococcus intermedius sont ensuite soumises à une double isoélectrofocalisation préparative.

Cette isoélectrofocalisation préparative est effectuée sur une colonne LKB 8100-1 de 110 ml. On utilise en premier lieu un gradient de kpH de 3,5 à 6 en mélangeant des proportions égales d'ampholytes de pH 3,5—5 (LKB 1809-111) et de pH 4—5 (LKB 4—6) de façon à avoir une concentration finale en ampholytes de 2% (poids/volume).

On effectue une seconde isoélectrofocalisation préparative avec un gradient de pH de 4—5 afin de séparer les fractions semipurifiées par la première isoélectrofocalisation. On utilise à cet effet des ampholytes ayant un pH de 4—5 (Ser-valyst) également d'une concentration finale en ampholytes de 2% (poids/volume).

La solution de l'anode, au fond, est constituée d'acide phosphorique à 1,0 M et la solution de la cathode au sommet, constituée d'hydroxyde de sodium à 1,0 M. De plus, le gradient de pH est stabilisé pour empêcher la convection à l'aide d'un gradient de densité verticale de saccharose réalisé en mélangeant une solution dense de saccharose (50% p/v) avec une solution légère (5% p/v) de saccharose en utilisant un mélangeur de gradient LKB 8121. Pratiquement, la colonne est remplie aux 2/3 de son volume et les échantillons sont mélangés avec la solution dense dans la proportion de 2/1 (v/v) et déposés avec précuation sur le sommet du gradient. Le reste de la solution de gradient est ensuite déposée au-dessus. Les échantillons sont constitués dans chaque cas de 3 préparations concentrées différentes de produits secrétés par Streptococcus intermedius ou de deux préparations de fraction semi-purifiée de ces préparations concentrées. Tous les échantillons sont reconstitués dans un volume de 3 à 6 ml après une nuit entière de dialyse à 4°C contre la glycine à 1% p/v.

Les conditions d'isoélectrofocalisation consistent en une tension constante de 1600 volts pendant 16 heures avec une intensité initiale de 10 mA, qui atteint une intensité terminale de 2 mA. Ensuite le voltage est augmenté jusqu'à 1800 volts pendant 4 heures de plus. Pendant l'expérience, la colonne est réfrigérée, la température étant de 2°C à l'entrée et de 5°C à la sortie de la colonne.

L'élution de la colonne est faite en poussant le gradient depuis le fond jusqu'au sommet grâce à l'injection d'eau distillée à l'aide d'une pompe péristaltique avec un débit d'approximativement 25 ml par heure. L'éluat est collecté en fractions de 1,5 ml. Le pH est mesuré à la température ambiante de chaque tube. Les fractions sont regroupées selon leur densité optique mesurée à 280 nm.

Les ampholytes et le saccharose sont retirés des fractions grâce à une dyalise contre un large volume d'une solution de glycine à 1%, puis par au moins deux étapes de dialyse sous vide. Quand la majeure partie du liquide est extrait du sac de dialyse, la solution résiduelle est mélangée à 50 volumes de milieu MEM (Gibco Diagnostics) et les échantillons sont de nouveau dialysés afin de ramener au même volume que l'échantillon initial qui a été fractionné.

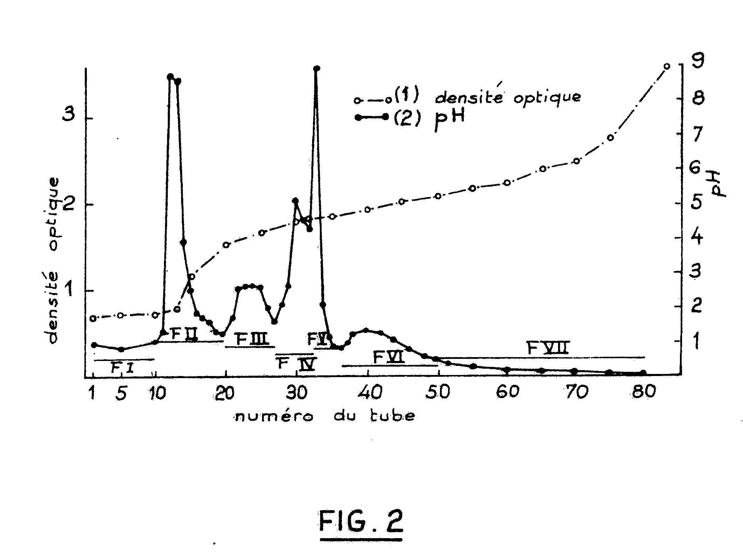

A l'issue du premier fractionnement, on a regroupé les fractions en 7 fractions principales (F I à F VIII) en fonction de leur densité optique. In a représenté sur la fig. 2 la densité optique des fractions et les 7 fractions principales regroupées. On à également reporté sur la fig. 2 le pH des fractions éluées.

L'activité immuno-suppressive a été trouvée dans les fractions F V (pH 4,52—4,68) et F VI (pH 4,70—5,25) mais principalement dans la fraction F VI, à la fois par les tests in vitro d'inhibition du captage de [³H]-thymidine par des cultures stimulées par PHA ou MLC et in vivo chez la souris.

Les fractions combinées F V + VI représentant environ 6% de la teneur totale en protéines de la préparation concentrée initiale.

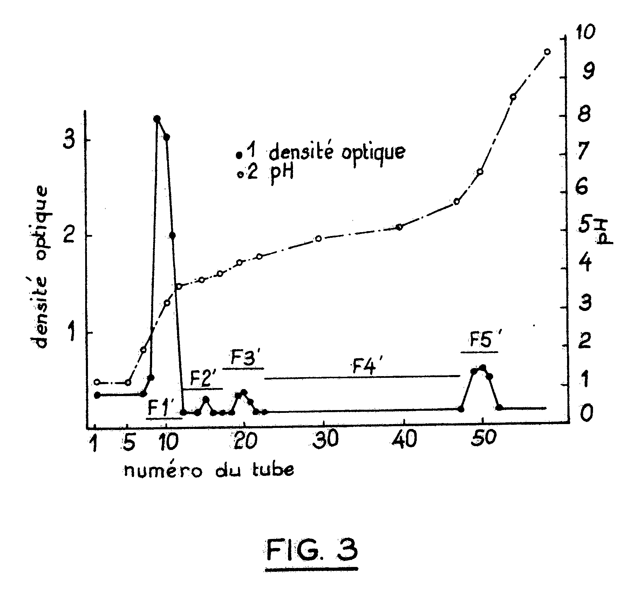

Les fractions combinées F V + VI ont été soumises à un second fractionnement par isoélectrofocalisation entre pH 4 et 5. On a représenté sur la fig. 3 la densité optique des fractions ainsi obtenues et le pH de ces fractions.

L'activité immuno-suppressive a été trouvée dans la fraction F 3' (pH 4,1—4,4). Cette fraction contient 15 µg/ml de protéine soit environ 1% de la concentration initiale en protéines.

L'activité immuno-suppressive a été observée dans les 4 tests (a, b, c et d) mentionnés ci-dessus.

On donnera dans le Tableau I ci-après les résultats obtenus dans les tests de captage de [³H]-thymidine de cultures stimulées par PHA et MLC.

Tableau I
Captage de [³H]-thymidine

| Concentration | PHA | MLC |
|---|---|---|
| 0 (témoin) | 35.821 ± 1 889 | 33.571 ± 1.169 |
| 1/400 | 56. ± 11 (0,15%) | 64 ± 19 (0,19%) |
| 1/800 | 75 ± 7 (0,21%) | 134 ± 11 (0,4%) |
| 1/1600 | 107 ± 15 (0,3%) | 171 ± 15 (0,5%) |
| 1/3200 | 1.074 ± 111 (3%) | 235 ± 11 (0,7%) |
| 1/6400 | 9.393 ± 396 (26%) | 2.014 ± 181 (6%) |

Les nombres entre parenthèses indiquent les valeurs moyennes de 3 essais, exprimées sous forme de pourcentage par rapport aux témoins.

On donnera dans le Tableau II ci-après les résultats obtenus dans le test d'immunisation de cellules mononucléaires humaines contre SRBC.

Tableau II

| Concentration | % par rapport aux témoins |
|---|---|
| 2 U MLC | 50 ± 2,5 |
| 4 | 25 ± 7,5 |
| 8 | 3 ± 1,3 |

*) U MLC = unités d'activité biologique MLC, c'est-à-dire quantité de substance produisant une réduction de 50% dans le captage de [³H]-thymidine stimulée par MLC.

Pour l'essai in vivo chez la souris, on a injecté une quantité de la fraction F 3' correspondant à 1 µg de protéine par voie intrapéritonéale à des souris, deux jours avant l'antigène constitué par des erythrocytes de mouton. On a obtenu une réponse correspondant à 5 ± 1,5% de celle obtenue pour les souris témoins n'ayant pas reçu d'injection de protéine.

La fraction F 3' a été soumise à une électrofocalisation analytique sur gel de polyacrylamide entre pH 4 et 6. On obtient une seule bande colorable au bleu de Coomasie. Cette bande a un point isoélectrique de 4,25. Cette bande n'est pas colorable au bleu de méthylène ni au PAS (méthode de détection des glycoprotéines utilisant de l'acide périodique et un réactif de Schiff).

Le spectre d'absorption UV entre 240 et 370 nm représenté sur la fig. 1, révèle un maximum d'absorption à 260 nm. La masse moléculaire de la substance présente dans la fraction F 3' a été estimée à 90 000 daltons, par mesure de la migration sur gels de polyacrylamide à 7,5% selon la méthode de Weber et Osborn (J. Biol. Chem. 244, 4406—4412), ainsi que par mesure de la mobilité selon la méthode de Thorun et Maurer (Disc electrophoresis and related techniques of polyacrylamide gel electrophoresis — Maurer, Walter de Gruyter Berlin Eds 8—31).

La substance présente dans la fraction F 3' est sensible à l'activité protéolytique des $\alpha$, $\delta$ et $\gamma$ chymotrypsines ainsi que de la trypsine. Elle est résistante à la désoxyribonucléase, à la ribonucléase A et à la neuraminidase. Elle est en revanche très sensible à la chaleur: elle est totalement détruite à 70° C après 30 min, partiellement détruite à 56° C après 30 min et très légèrement détruite à 37° C pendant 24 heures.

Ces éléments confirment la nature protéique de la substance isolée.

On a en outre constaté que cette protéine adhère au Sephadex® et à l'acétate de cellulose. Aussi, compte tenu de son maximum d'absorption à 260 nm on pense qu'elle contient de la phénylalanine parmi l'un de ses constituants principaux.

Il apparaît que la protéine isolée a une remarquable activité immuno-suppressive qui apparaît dans les différents tests utilisés à des doses très faibles, bien inférieures à la DL 50 par voie intrapéritonéale chez la souris qui est supérieure à 1 mg/kg.

Plus généralement, la substance selon l'invention peut être isolée des produits secrétés par les microorganismes suivants:

### a — Bactéries

Bactéries filamenteuses Gram +
  Actinomyces (A. israeli, A. viscosus,
    A. naeslundii, A. odontolyticus)
  Corynebactéries dont C. diphteriae,
    C. pyogenes, C. pseudotuberculosis,
    C. (Propionibacterium) acnes,
    G. Granulosum, C. parvum, C. vaginale
  Listeria dont L. monocytogenes
  Erysipelothrix rhusiopathiae
  Bacillus sont B. cereus
  Lactobacillus dont L. casei
  Clostridies sont C. perfringens

Bacilles acido-alcoolo résistants
  Mycobactéries sont M. Tuberculosis,
    M. bovis, M. avium, M. leprae.

Cocci Gram +
  Staphylocoques dont S. aureus,
    S. epidermididis.
  Streptocoques:
  S. pyogenes, S. mutans, S. mitis, S. sanguis,
    S. salivarius, S. intermedius et
    S. (Diplococcus) pneumoniae.
  Micrococcus sp et Diplococcus sp anaérobies.

  Bactéries filamenteuses Gram —
  Leptotrichia buccalis
  Fusobacterium (F. fusiforme, F. nucleatum)
  Bacteroides (B. melaninogenicus ssp. intermedius et melaninogenicus, B. oralis,
    B. asaccharolyticus)
  Bacillus (Capnocytophaga) ochraceus
  Campylobacter (Vibrio) fetus, C. coli,
    C. jejuni, C. sputorum
  Spirillum
  Eikenella corrodens
  Ramibacterium ramosum

Cocci Gram —
  Neisseria (N. gonorrhoeae, N. meningitidis,
    N. mucosa, N. perflava, N. subflava)
  Veillonella parvula
  Ristella fragilis
  Enterobactéries:
    Enterobacter, Serratia, Klebsiella, Citro-
  bacter,
    Escherichia (E. coli), Proteus, Providencia
  Pseudomonas dont P. aeruginosa
  Francisella tularensis
  Versinia (Y. pestis, Y. pseudotuberculosis,
    Y. enterocolitica)
  Haemophilus (H. influenzae, H. aegyptius,
    H. ducreyi)
  Brucella (B. melitensis, B. abortus, B. suis)
  Aeromonas

  Moraxella
  Bordetella (B. pertussis, B. bronchiseptica)
  Pasteurella (P. multocida, P. pneumotropica)
  Vibrio (V. cholerae, V. eltor)

Spirochetes
  Leptospira (L. icterohaemorrhagiae)
  Treponema (P. pallidum)
  Borrelia (B. recurrentis)

Mycoplasmes
  Mycoplasma (M. orale, M. salivarium)

Rickettsies
  Rickettsia (R. mooseri, R. prowazeki)
  Coxiella (C. burneti)

Chlamydiacées
  Chlamydia (C. trachomatis,
    C. lymphogralumatosis) (C. psittaci)

### b — Champignons

Candida (C. albicans)
Cryptococcus neoformans
Histoplasma caspulatum
Aspergillus fumi gatus

### c — Parasites

Protozoaires

  Amibes:
    Entamoeba (H. histolytica)
  Flagellés:
  — flagellés sanguicoles et S.R.E.
    Trypanosoma (T. gambiense, T. cruzi)
    Leischania (L. donovani, L. tropica)
  — flagelllés intestinaux et genito-urinaires
    Trichomonas vaginalis
    Giardia intestinalis
  Sporozoaires:
    Toxoplasma gondii
    Plasmodium (P. falciparum, P. vivax)
    Pneumocystis carinii

La substance selon l'invention peut être utilisée en thérapeutique comme immuno-suppresseur.

Elle trouve des applications, aussi bien en médecine humaine qu'en médecine vétérinaire, pour la prévention du rejet des organes transplantés, par exemple les transplantations rénales, le greffes de moëlle osseuse, les greffes cardiaques, ainsi que pour le traitement des maladies auto-immunes, par exemple le lupus erythémateux disséminé, la polyarthrite rhumatoïde, les syndrômes hématologiques auto-immuns, les syndrômes néphrotiques, pour le traitement des maladies à immun-complexes, des syndrômes inflammatoires, des affections rhumatologiques, des maladies du collagène, des manifestations allergiques, pour le traitement des affections neurologiques, odonto-stomatologiques respiratoires (ORL pulmonaires), digestives (maladie de Crohn, recto-colite hémorragique), urinaires et gynécologiques, pour le traitement des affections néoplasiques.

A cet effet, la substance selon l'invention peut être présentée sous diverses formes de compositions thérapeutiques, notamment sous forme d'une solution ou d'une suspension dans un soluté physiologique ou dans un excipient huileux ou encore d'une émulsion eau dans l'huile ou huile dans l'eau pour une administration intradermique, souscutanée, intramusculaire, intraveineuse, intrarachidienne, ou sous forme intégrée dans des particules lipidiques, sous forme lyophilisée, sous forme d'aérosols, sous forme buvable, sous forme de comprimés, dragées ou gélules pour une administration par voie orale, ou encore sous forme de suppositoires pour une administration rectale ou d'ovules pour une administration gynécologique.

Elle peut également se présenter sous forme de pommade, de spray ou de gel, dans un excipient adéquat, pour une administration topique.

Elle peut en général être administrée à l'homme à des doses de 0,1 à 10 mg.

## Revendications pour les Etats Contractants: BE, CH, LI, DE, FR, GB, IT, LU, NL, SE

1. Protéine ayant une activité immuno-suppressive et possédant les propriétés physico-chimiques suivantes:
(1) elle possède un point isoélectrique de 4,25;
(2) elle possède un spectre d'absorption ultraviolet, comme représenté sur la fig. 1, présentant un maximum à 260 nm;
(3) elle possède une masse moléculaire d'environ 90 000 daltons;
(4) elle est colorable au bleu de Coomasie; elle n'est pas colorable au bleu de méthylène ni au PAS;
(5) elle est résistante à la désoxyribonucléase, à la ribonucléase A et à la neuraminidase;
(6) elle est sensible aux $\alpha$, $\gamma$, $\delta$ chymotrypsines et à la trypsine;
(7) elle est totalement détruite à 70°C après 30 min, partiellement détruite à 56°C après 30 min et très légèrement détruite après incubation à 37°C pendant 24 heures;
(8) elle adhère au Séphadex® et à l'acétate de cellulose.
2. Procédé d'obtention de la protéine selon la revendication 1, caractérisé en ce que l'on soumet des produits secrétés par des microorganismes à une isoélectrofocalisation sur gradient de saccharose à pH 3,5—6, on sépare les fractions présentant une activité immuno-suppressive, on soumet ces fractions à une isoélectrofocalisation préparative sur gradient de saccharose à pH 4—5 et l'on isole la fraction présentant une activité immuno-suppressive.
3. Procédé selon la revendication 2, caractérisé en ce que l'on utilise comme matières de base les produits secrétés par Streptococcus intermedius.
4. Composition thérapeutique ayant une activité immuno-suppressive, caractérisée en ce qu'elle contient à titre de principe actif une protéine selon la revendication 1.

## Revendications pour l'Etat Contractant: AT

1. Procédé d'obtention d'une protéine ayant une activité immuno-suppressive et possédant les propriétés physico-chimiques suivantes:
(1) elle possède un point isoélectrique de 4,25;
(2) elle possède un spectre d'absorption ultraviolet, comme représenté sur la fig. 1, présentant un maximum à 260 nm;
(3) elle possède une masse moléculaire d'environ 90 000 daltons;
(4) elle est colorable au bleu de Coomasie; elle n'est pas colorable au bleu de méthylène ni au PAS;
(5) elle est résistante à la désoxyribonycléase, à la ribonucléase A et à la neuraminidase;
(6) elle est sensible aux $\alpha$, $\gamma$, $\delta$ chymotrypsines et à la trypsine;
(7) elle est totalement détruite à 70°C après 30 min, partiellement détruite à 56°C après 30 min et très légèrement détruite après incubation à 37°C pendant 24 heures;
(8) elle adhère au Séphadex® et à l'acétate de cellulose,
caractérisé en ce qu'on soumet des produits secrétés par des microorganismes à une isoélectrofocalisation sur gradient de saccharose à pH 3,5—6, on sépare les fractions présentant une activité immuno-suppressive, on soumet ces fractions à une isoélectrofocalisation préparative sur gradient de saccharose à pH 4—5 et on isole la fraction présentant une activité immuno-suppressive.
2. Procédé selon la revendication 1, caractérisé en ce qu'on utilise comme matières de base les produits secrétés par Streptococcus intermedius.
3. Procédé de préparation d'une composition thérapeutique ayant une activité immuno-suppressive, caractérisé en ce qu'on met une protéine définie à la revendication 1 sous une forme thérapeutiquement administrable.

## Patentansprüche für die Vertragsstaaten: BE, CH, LI, DE, FR, GB, IT, LU, NL, SE

1. Protein mit immunosuppressiver Wirkung und den folgenden physiko-chemischen Eigenschaften:
(1) es besitzt einen isoelektrischen Punkt von 4,25;
(2) es besitzt ein UV-Absorptionsspektrum wie in F i g. 1 dargestellt mit einem Maximum bei 260 nm;
(3) es besitzt eine Molmasse von ungefähr 90 000 Dalton;

(4) es ist mit Coomasie-Blau färbbar; es ist weder mit Methylen-Blau noch mit PAS färbbar;

(5) es ist gegenüber Desoxyribonuclease, Ribonuclease A und Neuraminidase resistent;

(6) es ist empfindlich gegenüber $\alpha$, $\gamma$, $\delta$-Chymotrypsinen und gegenüber Trypsin;

(7) es wird total zerstört bei 70° C nach 30 min, teilweise zerstört bei 56° C nach 30 min und sehr leicht zerstört nach Inkubation bei 37° C während 24 h;

(8) es haftet an Sephadex® und an Celluloseacetat.

2. Verfahren zur Herstellung des Proteins nach Anspruch 1, dadurch gekennzeichnet, daß man von Mikroorganismen ausgeschiedene Produkte einer Isoelektrofokussierung auf einem Saccharose-Gradienten bei pH 3,5 bis 6 unterwirft, die eine immunosuppressive Wirkung aufweisenden Fraktionen abtrennt, diese Fraktionen einer präparativen Isoelektrofokussierung auf Saccharose-Gradient bei pH 4 bis 5 unterwirft und die eine immuno-suppressive Wirkung aufweisende Fraktion isoliert.

3. Verfahren nach Anspruch 2, dadurch gekennzeichnet, daß man als Ausgangsstoffe die durch Streptococcus intermedius ausgeschiedenen Produkte verwendet.

4. Therapeutische Zusammensetzung mit einer immunosuppressiven Wirkung, dadurch gekennzeichnet, daß sie als Wirkstoffe ein Protein nach Anspruch 1 enthält.

**Patentansprüche für den Vertragsstaat: AT**

1. Verfahren zur Herstellung eines Proteins mit einer immunosuppressiven Wirkung und den folgenden physiko-chemischen Eigenschaften:

(1) es besitzt einen isoelektrischen Punkt von 4,25;

(2) es besitzt ein UV-Absorptionsspektrum wie in Fig. 1 dargestellt mit einem Maximum bei 260 nm;

(3) es besitzt eine Molmasse von ungefähr 90 000 Dalton;

(4) es ist mit Coomasie-Blau färbbar; es ist weder mit Methylen-Blau noch mit PAS färbbar;

(5) es ist gegenüber Desoxyribonuclease, Ribonuclease A und Neuraminidase resistent;

(6) es ist empfindlich gegenüber $\alpha$, $\gamma$, $\delta$-Chymotrypsinen und gegenüber Trypsin;

(7) es wird total zerstört bei 70° C nach 30 min, teilweise zerstört bei 56° C nach 30 min und sehr leicht zerstört nach Inkubation während 37° C während 24 h;

(8) es haftet an Sephadex® und an Celluloseacetat,

dadurch gekennzeichnet, daß man von Mikroorganismen ausgeschiedene Produkte einer Isoelektrofokussierung auf einem Saccharose-Gradienten bei pH 3,5 bis 6 unterwirft, die eine immunosuppressive Wirkung aufweisenden Fraktionen abtrennt, diese Fraktionen einer präparativen Isoelektrofokussierung auf Saccharose-Gradient bei pH 4 bis 5 unterwirft und die eine immunosuppressive Wirkung aufweisende Fraktion isoliert.

2. Verfahren nach Anspruch 1, dadurch gekennzeichnet, daß man als Ausgangsstoffe die durch Streptococcus intermedius ausgeschiedenen Produkte verwendet.

3. Verfahren zur Herstellung einer therapeutischen Zusammensetzung mit einer immunosuppressiven Wirkung, dadurch gekennzeichnet, daß man ein gemäß Anspruch 1 definiertes Protein in eine therapeutisch anwendbare Form bringt.

**Claims for the Contracting States: BE, CH, LI, DE, FR, GB, IT, LU, NL, SE**

1. Protein having an immunosuppressive activity and the following physico chemical properties:

(1) its isoelectric point is 4.25;

(2) it has an ultraviolet absorption spectrum, as shown in fig. 1, with a maximum at 260 nm;

(3) its molecular mass is about 90 000 daltons;

(4) it can be stained by Coomasie blue, but neither by Methylene blue nor PAS;

(5) it is desoxyribonuclease, ribonuclease A and neuraminidase proof;

(6) it is degraded by $\alpha$, $\gamma$, $\delta$ chymotrypsins and trypsin;

(7) it is thorough destroyed at 70° C after 30 mn, partially destroyed at 56° C after 30 mn and very slightly destroyed after incubation at 37° C for 24 hours;

(8) it adheres to Sephadex® and cellulose acetate.

2. Process for the production of a protein according to claim 1, characterized by subjecting the products secreted by microorganisms to a preparative isoelectrofocusing on a saccharose gradient at pH 3.5—6, separating the fractions with immunosuppressive activity, subjecting these fractions to a preparative isolectrofocusing on a saccharose gradient at pH 4—5 and isolating the fraction with immunosuppressive activity.

3. Process according to claim 2, characterized by using products secreted by Streptococcus Intermedius.

4. Therapeutic composition having an immunosuppressive activity characterized in that it contains as active ingredient a protein as claimed in claim 1.

**Claims for the Contracting State: AT**

1. Process for the production of a protein having an immunosuppressive activity and the following physico chemical properties:

(1) its isoelectric point is 4.25;

(2) it has an ultraviolet absorption spectrum, as shown in fig. 1, with a maximum at 260 nm;

(3) its molecular mass is about 90 000 daltons;

(4) it can be stained by Coomasie blue, but neither by Methylene blue nor PAS;

(5) it is desoxyribonuclease, ribonuclease A and neuraminidase proof;

(6) it is degraded by $\alpha$, $\gamma$, $\delta$ chymotrypsins and trypsin;

(7) it is thorough destroyed at 70°C after 30 mn, partially destroyed at 56°C after 30 mn and very slightly destroyed after incubation at 37°C for 24 hours;

(8) it adheres to Sephadex® and cellulose acetate,

characterized by subjecting the products secreted by microorganisms to a preparative isoelectrofocusing on a saccharose gradient at pH 3.5—6, separating the fractions with immunosuppressive activity, subjecting these fractions to a preparative isoelectrofocusing on a saccharose gradient at pH 4—5 and isolating the fraction with immunosuppressive activity.

2. Process according to claim 1, characterized by using products secreted by Streptococcus Intermedius.

3. Process for the preparation of a therapeutic composition having an immunosuppressive activity characterized in that a protein as defined in claim 1 is put under a therapeutically administrable form.

FIG. 1

FIG. 2

FIG. 3

0 045 237